# EUROPÄISCHE PATENTANMELDUNG

(11) **EP 1 576 948 A2**
(43) Veröffentlichungstag der Anmeldung: **21.09.2005**
(21) Anmeldenummer: 05101298.7
(22) Anmeldetag: 21.02.2005
(51) Int. Cl.: A61K 7/48, A61K 7/50

(54) **Wasserfreie Reinigungszubereitungen mit polaren Farbstoffen**

(30) Priorität: 17.03.2004 DE 102004013382
(71) Anmelder: Beiersdorf AG, 20245 Hamburg (DE)
(72) Erfinder: Bluschke, Torsten, 21717 Fredenbeck-Schwinge (DE); Kuether, Jörg, 25469 Halstenbek (DE)

(57) **Zusammenfassung**

Ölhaltige, wasserfreie kosmetische Zubereitung enthaltend:
mehr als 45 %, bevorzugt mehr als 60%, besonders bevorzugt mehr als 75%, ganz besonders bevorzugt mehr als 85% ÖI,
0,00001- 5 % eines polaren Farbstoffes mit einem n-Octanol/Wasser Verteilungskoeffizienten von weniger als 1 dadurch gekennzeichnet, dass sie einphasig ist und beim stehen lassen innerhalb von 24 Stunden keine Phasenseperation auftritt.

## Beschreibung

Die vorliegende Erfindung betrifft durch polare Farbstoffe eingefärbte, wasserfreie, tensidhaltige Reinigungsformlierungen, insbesondere langzeitstabile tensid- und ölhaltige Zubereitungen.

Wasserfrei im Sinne der vorliegenden Schrift ist eine Zubereitung mit weniger als 1 Gew.% Wasser.

Tensid- und ölhaltige Zubereitungen im Sinne der vorliegenden Schrift sind beispielsweise Ölbäder, Duschöle, Pflegeöle.

Langzeitstabil im Sinne der vorliegenden Schrift ist eine Zubereitung, die länger als 24 Stunden gelagert werden kann, ohne einer Phasenseparation zu unterliegen, in der sich polare und unpolare Phase trennen.

Polar im Sinne der vorliegenden Schrift ist ein Stoff, der einen Verteilungskoeffizienten in einer n-Octanol/Wasser Mischung aufweist, der einen Wert von kleiner eins ist.

Die bekannten Produkte werden durch öllösliche Farbstoffe eingefärbt und sind deshalb nahezu immer sehr ähnlich gefärbt. Sie weisen zumeist grüne oder violette Einfärbungen auf. Zur Einfärbung wird ein Farbstoff verwendet. Es gibt aber nur sehr wenige Farbstoffe, die in Kosmetika ohne Beschränkungen eingesetzt werden dürfen und die öllöslich sind. Ohne einen solchen Farbstoff entspricht die Farbe des finalen Produktes der der verwendeten Rohstoffe. Daher sind die farblichen Gestaltungsmöglichkeiten für Produkte dieser Art sehr eingeschränkt.

Gern würde man die Gestaltungsmöglichkeiten nutzen, die polare Farbstoffe ermöglichen. Es gibt eine große Zahl für den Einsatz in Kosmetika zugelassener polarer Farbstoffe. Um diese in vorwiegend aus Öl bestehenden Zubereitungen einsetzen zu können, benötigt man aber größere Mengen Wasser, in der Regel mehr als 5 Gew.%. Übersteigt aber der Wassergehalt einer überwiegend aus Tensiden und Ölen bestehenden Zubereitungen den Wert von 3 bis 4 Gew.%, zukommt es rasch zu einer Phasetrennung und die Zubereitung ist nicht lange Zeit lagerstabil. Oft erhält man auf diese Weise trübe Zubereitungen. Die Trübung einer Tensid- und ölhaltigen Zubereitungen ist aber unerwünscht: derartige Produkte werden vom Verbraucher weitgehend abgelehnt.

Davon ausgehend lag der vorliegenden Erfindung die Aufgabe zugrunde, ölhaltige und dabei wasserfreie Rezepturen zu finden, die polare Farbstoffe enthalten. Es hat sich für den Fachmann nicht vorhersehbar herausgestellt, daß ölhaltige, wasserfreie kosmetische Zubereitung enthaltend: mehr als 45 %, bevorzugt mehr als 60%, besonders bevorzugt mehr als 75%, ganz besonders bevorzugt mehr als 85% Öl, 0,00001- 5 % eines polaren Farbstoffes mit einem n-Octanol/Wasser Verteilungskoeffizienten von weniger als 1 dadurch gekennzeichnet, dass sie einphasig ist und beim stehen lassen innerhalb von 24 Stunden keine Phasenseperation auftritt, den Mängeln des Standes der Technik abhelfen.

Durch Verwendung spezieller Rezepturen ist es gelungen durch polare Farbstoffe Ölrezepturen ohne Ausbildung einer Phasentrennung langzeitstabil gleichmäßig einzufärben. Dadurch werden für diesen Formeltyp Farbeinstellungen möglich die bisher nicht denkbar waren.

Durch Entwicklung von speziellen Formulierungen gelingt es polare Farbstoffe ohne Phasentrennung langzeitstabil in oben beschriebene Formulierugen einzuarbeiten.

Während der Anwendung in der Badewanne bildet sich auf dem Badewasser ein dünner pflegender Ölfilm, welcher die Rückfettung der Haut sicherstellt. In den bisher bekannten und mit unpolaren Farbstoffen angefärbten Formulierungen befindet sich der Farbstoff in diesem oberen Film und zieht beim Heraussteigen aus der Wanne auf die Haut auf. Ein Großteil davon findet sich dann nach dem Abtrocknen im Badetuch wieder und verfärbt dieses. Ein weiterer verbraucherrelevanter Vorteil dieser Rezepturen besteht deshalb darin, dass sich der Farbstoff in der Wanne in der Wasserphase befindet und nur sehr wenig davon nach dem Bad auf die Haut übertragen wird. Es kann also im Normalfall nicht zu Verfärbungen von Textilien kommen.

Es wurde weiter gefunden, daß es bevorzugt ist, wenn eine solche Zubereitung wenigstens 3 % Tensid oder Tenside enthält. Dabei ist es bevorzugt, wenn das oder die Tenside anionische Tenside darstellen. Ganz besonders bevorzugt ist es, wenn der Gehalt an Tensid oder von Tensiden 5 bis 50 Gew.% beträgt.

Weiter ist es bevorzugt, wenn der Wassergehalt weniger als 1 Gew.% beträgt. Dabei ist es besonders bevorzugt, wenn der Wassergehalt 0 bis 0,15 Gew.%, ganz außergewöhnlich bevorzugt 0,01 bis 0,1 Gew.% beträgt. Verzichtet man ganz auf einen Wassergehalt, so kann je nach Farbstoff oder Farbstoffmischung ein Teil des Farbstoffes ungelöst bleiben.

Weiter ist es bevorzugt, wenn eine solche Zubereitung zusätzlich Emulgatoren enthält.

Weiter ist es bevorzugt, wenn die Zubereitung transparent und klar ist.

Bei all dem ist es bevorzugt, wenn der Gehalt an Farbstoff 0,00001 bis 0,01 Gew.% beträgt. Besonders bevorzugt ist es, wenn eine erfindungsgemäße Zubereitung mindestens einen Farbstoff mit einem Farbindex (Color Index, C.I.) gewählt aus C.I. 11680, 11680, 12490, 13015, 14720, 15985, 16035, 16255, 17200, 19140, 28440, 42051, 42090, 45170, 47005, 47005, 61570, 14720, 42053 enthält.

Die Erfindung umfasst auch die Verwendung einer Zubereitung nach einem der vorangehenden Ansprüche zur Verringerung der Verfärbung von Textilien nach der Anwendung am Körper sowie ein Verfahren zur Herstellung einer Zubereitung nach einem der vorangehenden Ansprüche umfassend die Schritte
(a) Herstellung einer sämtliche Ölkomponenten enthaltenen Ölphase,
(b) Herstellung einer Wirkstoffphase mit einem Gehalt an einem oben beschriebenen Farbstoff,
(c) Vereinigung der Wirkstoffphase - und Ölphase bei Raumtemperatur unter Rühren,
(d) Eventuelle Zugabe von mindestens einem oben beschriebenen Emulgator.

Weiter umfasst die Erfindung die Verwendung von solchen Zubereitungen als Ölbad, Duschöl, oder Pflegeöl. Unter anderem bei einer solchen Anwendung bilden die beschriebenen Zubereitungen bei Kontakt mit größeren Mengen Wasser eine milchige, weiße Emulsion bilden, beispielsweise, wenn der Überschuss an Wasser größer als 100 bezogen auf die ölhaltige Zubereitung ist.

Erfindungsgemäß bevorzugte polare Farbstoffe sind:

| **C.I.** | **Internat. Bez.** |
|---|---|
| 11680 | Yellow-P/2299 |
| 12490 | Red-P/2241 |
| 13015 | Yellow-W/1144 |
| 14720 | Red-W/1209 |
| 15985 | Orange-W/12541 |
| 16035 | Red-W/12100 |
| 16255 | Red-W/12132 |
| 17200 | Red-W/12052 |
| 19140 | Yellow-W/12254 |
| 28440 | Black-W/12535 |
| 42051 | Blue-W/11518.9 |
| 42090 | Blue-W/11509 |
| 45170 | Red-W/12255 |
| 47005 | Yellow-W/11847 |
| 47005 | Yellow-W/12270 |
| 61570 | Green-W/11404 |
| 14720 | Red-W/13149 |
| 42053 | Green-W/13144 |

Erfindungsgemäß bevorzugte Emulgatoren sind Fettalkohole und / oder deren Derivtate wie beispielsweise Laureth-2, Laureth-4, Trilaureth-4 Phosphat oder Lanolin Alkohol.

Erfindungsgemäß bevorzugte Tenside sind öllösliche Tenside oder Tensidmischungen wie beispielsweise MIPA Lauthsulfat, TIPA Laurethsulfat, Cocamide MEA, Cocamid DEA, Soyamid MEA, Isosteramid MEA.

Die Ölkomponenten gemäß der vorliegenden Erfindung werden vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie besonders bevorzugt der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Distelöl, Sojaöl, Erdnussöl, Rapsöl, Reisöl, Mohnöl, Sanddornöl, Traubenkernöl, Weizenkeimöl, Walnussöl, Pfirsichkernöl, Kürbiskernöl, Nachtkerzenöl, Mandelöl, Palmöl, Kokosöl, Palmkernöl und dergleichen mehr.

Es ist auch von Vorteil, den erfindungsgemäßen Zubereitungen Antioxidantien zuzusetzen. Vorteilhaft werden die Antioxidantien gewählt aus der Gruppe der öllöslichen Antioxidantien, insbesondere Tocopherol und/oder seine Derivate.

Weiterhin kann die beschriebene Zubreitung vorteilsweise Pflanzenextrakte enthalten. Dieses können sowohl alkoholische als auch wässerige Auszüge von Pflanzen umfassen. Dies umfasst beispielsweise Gewürzextrakte, Blumenextrakte, Holunderextrakt, Tannennadelextrakte, Algenextrakte, Lilienextrakt, Bambusextrakt, Honigextrakt, und andere mehr.

Auch öllösliche UV-Filtersubstanzen sind vorteilhaft, beispielsweise: 3-Benzylidencampher-Derivate, vorzugsweise 3-(4-Methylbenzyliden)campher, 3-Benzylidencampher; 4-Aminobenzoesäure-Derivate, vorzugsweise 4-(Dimethylamino)-benzoesäure(2-ethylhexyl)ester, 4-(Dimethylamino)benzoesäureamylester; 2,4,6-Trianilino-(p-carbo-2'-ethyl-1'-hexyloxy)-1 ,3,5-triazin;Ester der Benzalmalonsäure, vorzugsweise 4-Methoxybenzalmalonsäuredi(2-ethylhexyl)ester; Ester der Zimtsäure, vorzugsweise 4-Methoxyzimtsäure(2-ethylhexyl)ester, 4-Methoxyzimtsäureisopentylester;Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie an Polymere gebundene UV-Filter, bestimmte Salicylsäurederivate wie 4-Isopropylbenzylsalicylat, 2-Ethylhexylsalicylat (= Octylsalicylat), Homomenthylsalicylat. 1-(4'-tert.Butylphenyl)-3-(4'-methoxyphenyl)propan-1,3-dion und 1-Phenyl-3-(4'-isopropylphenyl)propan-1,3-dion.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

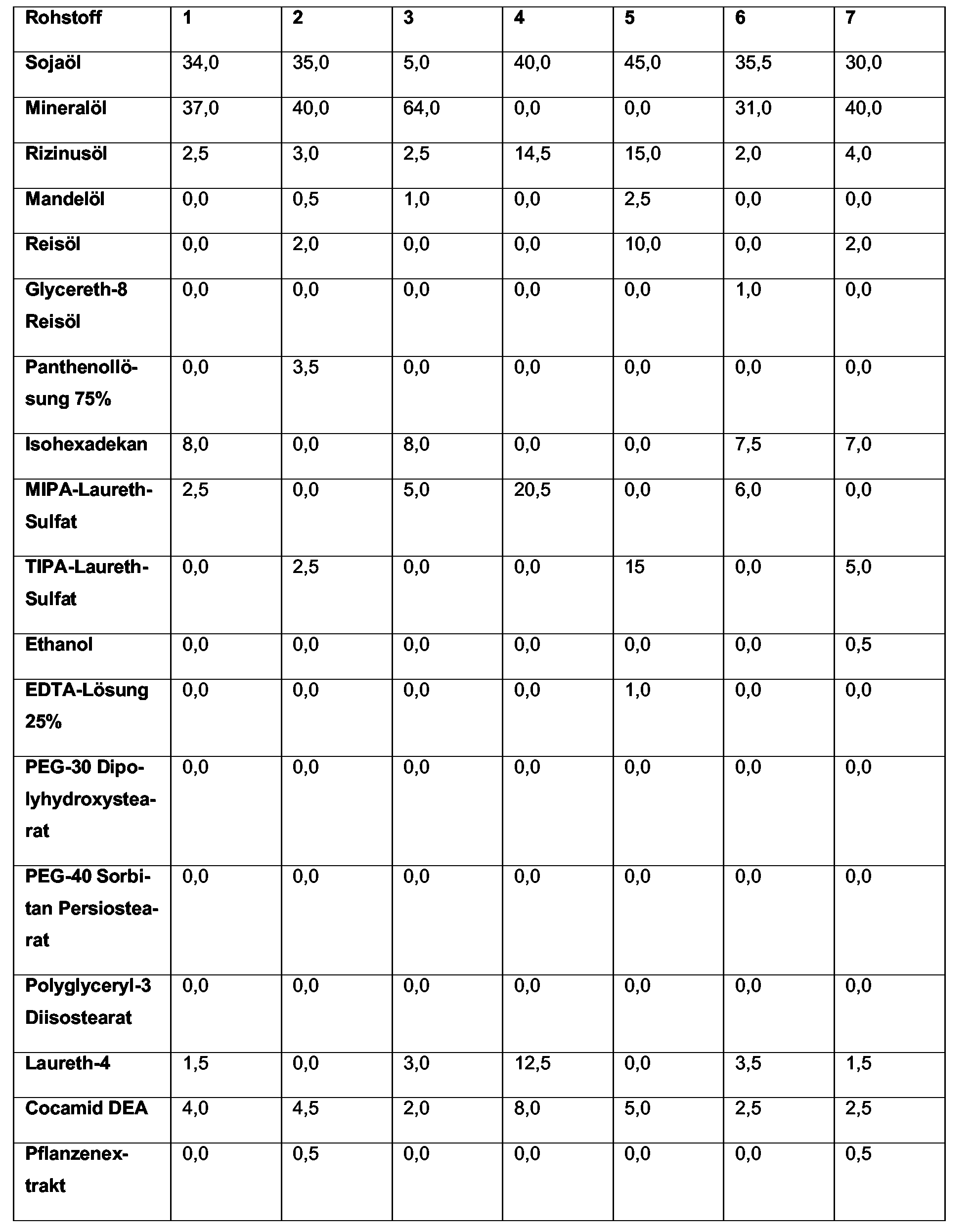

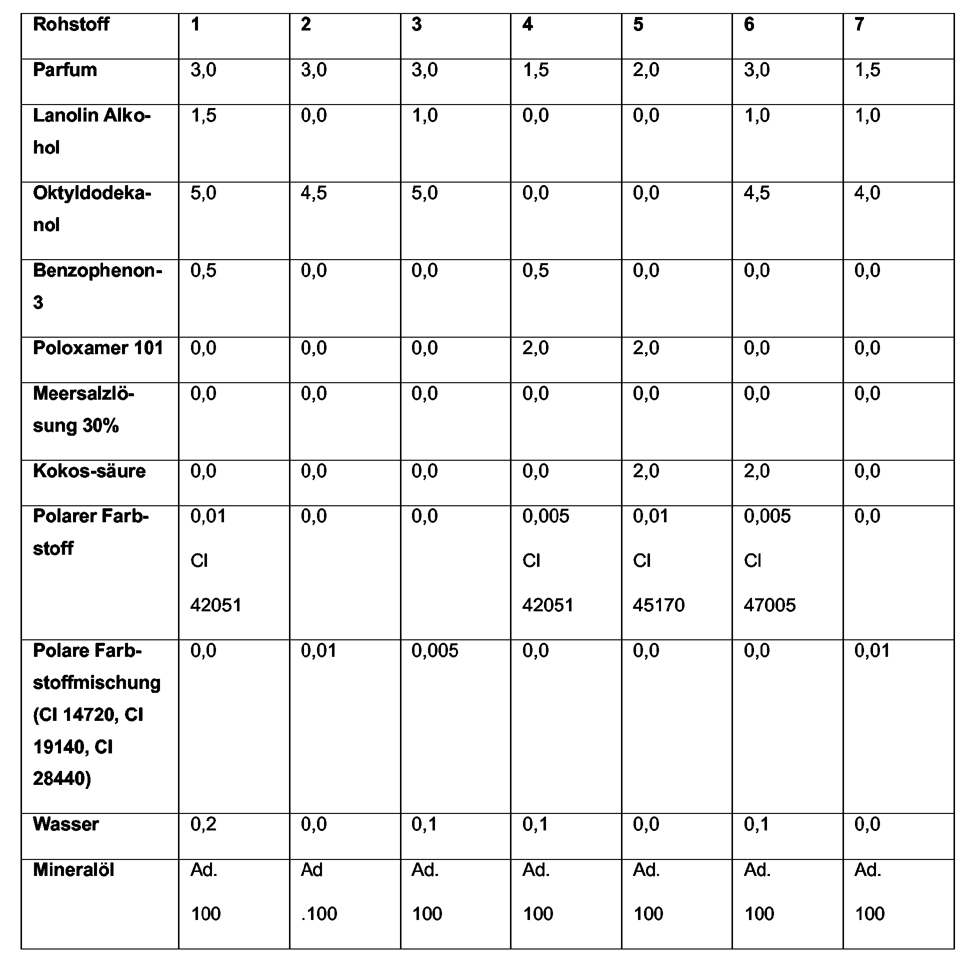

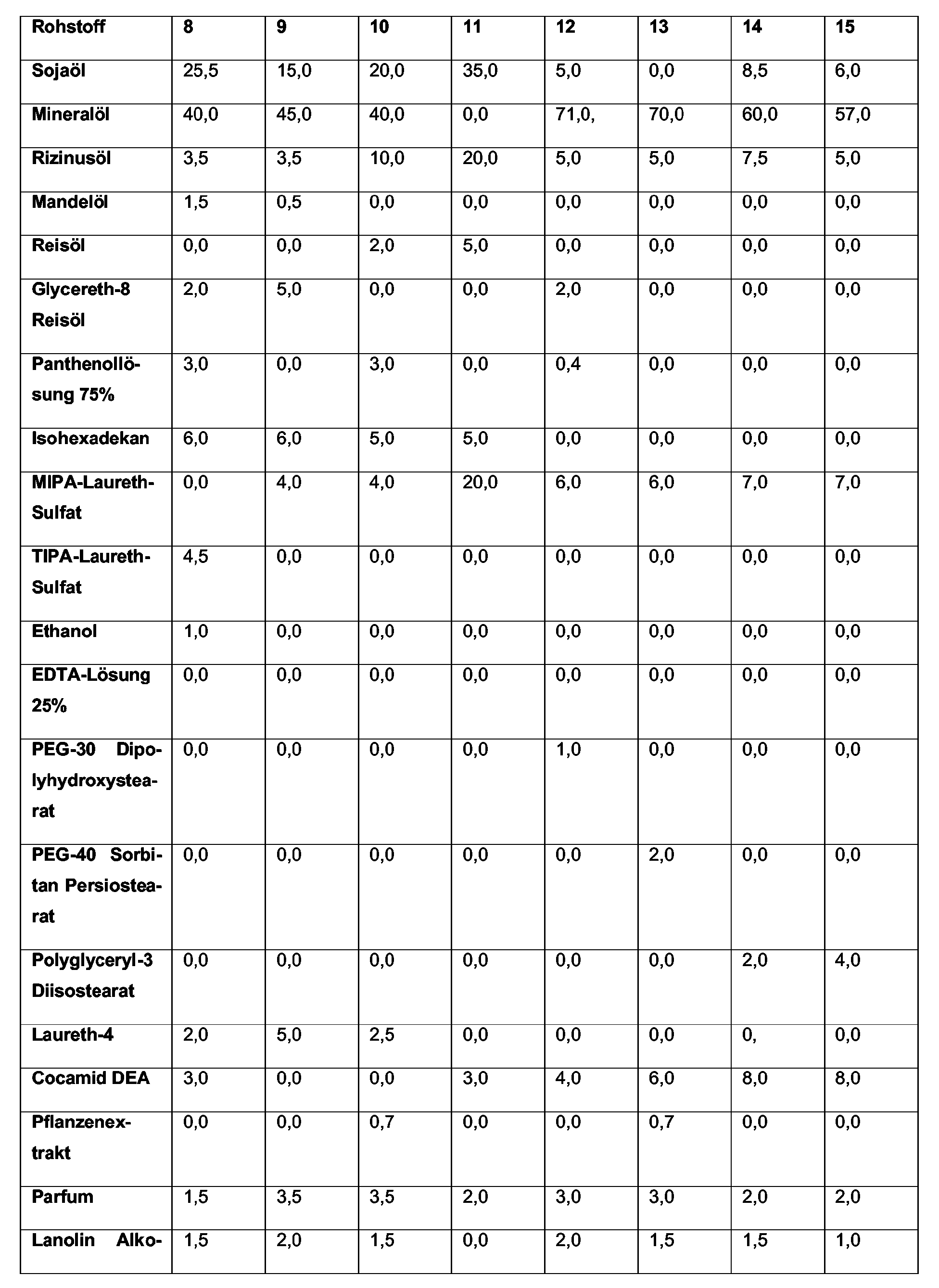

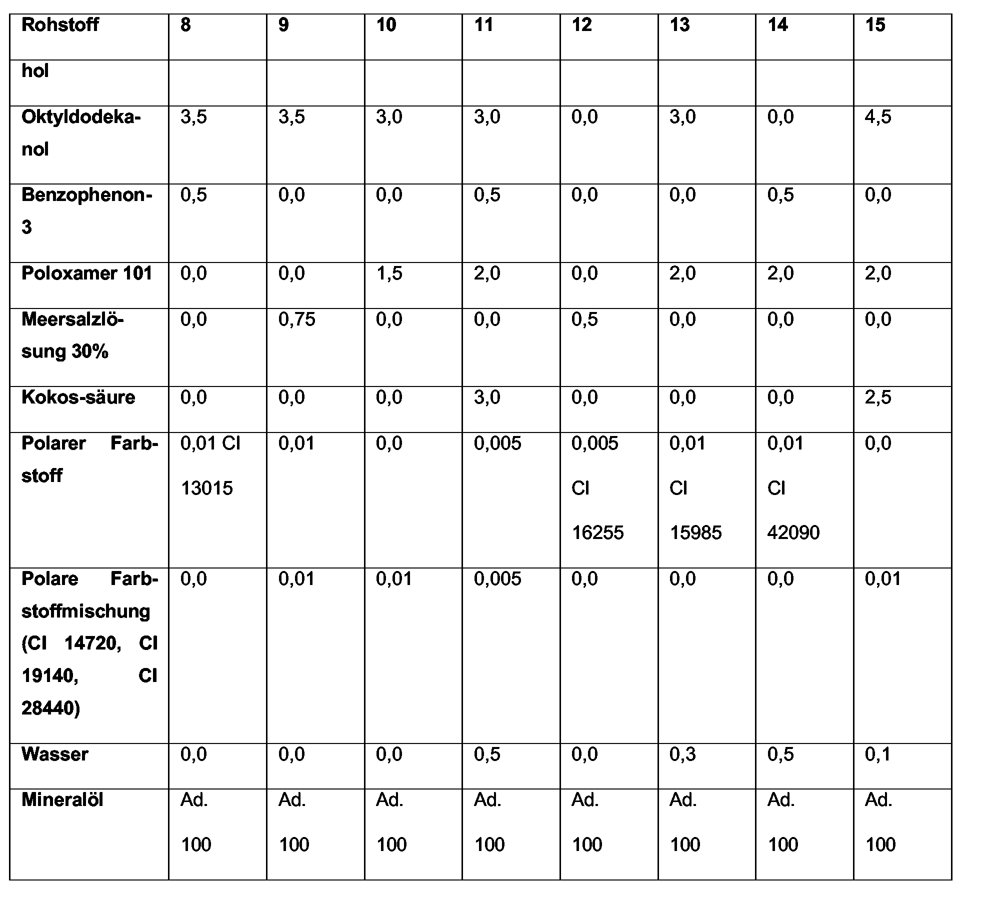

## Patentansprüche

1. Ölhaltige, wasserfreie kosmetische Zubereitung enthaltend:
mehr als 45 %, bevorzugt mehr als 60%, besonders bevorzugt mehr als 75%,
ganz besonders bevorzugt mehr als 85% Öl, 0,00001- 5 % eines polaren Farbstoffes mit einem n-Octanol/Wasser Verteilungskoeffizienten von weniger als 1 **dadurch gekennzeichnet, dass** sie einphasig ist
und beim stehen lassen innerhalb von 24 Stunden keine Phasenseperation auftritt.

2. Zubereitung nach Anspruch 1 enthaltend wenigstens 3 % Tensid oder Tenside.

3. Zubereitung nach Anspruch 2 **dadurch gekennzeichnet, dass** das oder die Tenside anionische Tenside darstellen.

4. Zubereitung nach einem der vorangehenden Ansprüche **dadurch gekennzeichnet, dass** der Gehalt an Tensid oder von Tensiden 5 bis 50 Gew.% beträgt.

5. Zubereitung nach einem der vorangehenden Ansprüche **dadurch gekennzeichnet, dass** der Wassergehalt weniger als 1 Gew.% beträgt.

6. Zubereitung nach einem der vorangehenden Ansprüche **dadurch gekennzeichnet, dass** der Wassergehalt 0 bis 0,15 Gew. % beträgt.

7. Zubereitung nach einem der vorangehenden Ansprüche **dadurch gekennzeichnet, dass** sie zusätzlich Emulgatoren enthält.

8. Zubereitung nach einem der vorangehenden Ansprüche **dadurch gekennzeichnet, dass** die Zubereitung transparent und klar ist.

9. Zubereitung nach einem der vorangehenden Ansprüche **dadurch gekennzeichnet, dass** der Gehalt an Farbstoff 0,001 bis 0,01 Gew.% beträgt.

10. Zubereitung nach einem der vorangehenden Ansprüche **dadurch gekennzeichnet, dass** sie mindestens einen Farbstoff mit einem Farbindex (Color Index, C.I.) gewählt aus C.I. 11680, 11680, 12490, 13015, 14720, 15985, 16035, 16255, 17200, 19140, 28440, 42051, 42090, 45170, 47005, 47005, 61570, 14720, 42053 enthält.

11. Verwendung einer Zubereitung nach einem der vorangehenden Ansprüche zur Verringerung der Verfärbung von Textilien nach der Anwendung am Körper.

12. Verfahren zur Herstellung einer Zubereitung nach einem der vorangehenden Ansprüche umfassend die Schritte
(a) Herstellung einer sämtliche Ölkomponenten enthaltenen Ölphase,
(b) Herstellung einer Wirkstoffphase mit einem Gehalt an Farbstoff nach einem der vorangehenden Ansprüche,
(c) Vereinigung der Wirkstoffphase - und Ölphase bei Raumtemperatur unter Rühren,
(d) Eventuelle Zugabe von Emulgatoren nach einem der vorangehenden Ansprüche.

13. Verwendung von Zubereitungen nach einem der vorangehenden Ansprüche als Ölbad, Duschöl, oder Pflegeöl.

14. Zubereitungen nach einem der vorangehenden Ansprüche die bei Kontakt mit größeren Mengen Wasser eine milchige, weiße Emulsion bilden.
